(19) Europäisches Patentamt
European Patent Office
Office européen des brevets

(11) **EP 3 755 386 B1**

(12) **FASCICULE DE BREVET EUROPEEN**

(45) Date de publication et mention de la délivrance du brevet:
**13.09.2023 Bulletin 2023/37**

(21) Numéro de dépôt: **19711975.3**

(22) Date de dépôt: **20.02.2019**

(51) Classification Internationale des Brevets (IPC):
**A61L 15/58** (2006.01)    **A61L 24/06** (2006.01)
**C09J 133/08** (2006.01)

(52) Classification Coopérative des Brevets (CPC):
(C-Sets disponibles)
**C09J 133/08; A61L 15/58**                    (Cont.)

(86) Numéro de dépôt international:
**PCT/FR2019/050386**

(87) Numéro de publication internationale:
**WO 2019/162613 (29.08.2019 Gazette 2019/35)**

(54) **COMPOSITION PRÉSENTANT UNE EXCELLENTE PERMÉABILITÉ À LA VAPEUR D'EAU**

ZUSAMMENSETZUNG MIT AUSGEZEICHNETER WASSERDAMPFDURCHLÄSSIGKEIT

COMPOSITION HAVING EXCELLENT WATER VAPOR PERMEABILITY

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **20.02.2018 FR 1851443**

(43) Date de publication de la demande:
**30.12.2020 Bulletin 2020/53**

(73) Titulaire: **URGO RECHERCHE INNOVATION ET DEVELOPPEMENT**
**21300 Chenôve (FR)**

(72) Inventeurs:
• **DANÉROL, Anne-Sophie**
**21000 DIJON (FR)**
• **GUILLAMAUD, Christelle**
**21300 CHENÔVE (FR)**

• **PERNOT, Jean-Marc**
**21000 DIJON (FR)**

(74) Mandataire: **Plasseraud IP**
**66, rue de la Chaussée d'Antin**
**75440 Paris Cedex 09 (FR)**

(56) Documents cités:
**WO-A1-2015/192122    US-A- 6 080 797**

(52) Classification Coopérative des Brevets (CPC):
(Cont.)

C-Sets
**A61L 15/58, C08L 33/08;**
**C09J 133/08, C08L 33/02**

**Description**

**RESUME**

La présente invention est relative à une nouvelle composition comprenant au moins un adhésif acrylique et des particules d'un polymère réticulé ayant une densité de groupement carboxylate comprise entre 2,0 et 12,0 meq/g et une taille de pore moyenne comprise entre 0,005 et 1,0 $\mu$m, utilisable notamment pour la réalisation d'une matrice adhésive convenant à tout dispositif, par exemple à visée médicale tel qu'un patch, un film, une bande ou un pansement, de préférence un pansement, ou bien encore convenant à tout dispositif de type textile fonctionnel tel qu'un article de sport.

La présente invention a trait tout particulièrement à une composition présentant une excellente perméabilité à la vapeur d'eau. Cette composition est notamment capable de mettre en oeuvre cette excellente propriété dans des domaines techniques aussi variés que le domaine médical, les emballages agro-alimentaires, l'automobile, les articles techniques de sport ou de loisir ou encore le domaine du textile.

**ETAT DE LA TECHNIQUE ANTERIEURE**

Un pansement est assimilable notamment à un dispositif de protection permettant de recouvrir une plaie. Un pansement peut avoir plusieurs autres fonctions cumulables entre elles ou non, telles que:

- protéger la plaie et l'isoler du milieu extérieur ;
- permettre une meilleure cicatrisation en maintenant un milieu humide favorable sur le lit de la plaie ;
- faire cesser un saignement minime ;
- rapprocher les berges d'une plaie ;
- absorber et gérer les exsudats afin de préserver les berges de la plaie et la peau péri-lésionnelle.

La cicatrisation naturelle d'une plaie se déroule en trois phases successives, chacune de ces phases étant caractérisée par des activités cellulaires spécifiques et différentes : la phase de détersion, la phase de bourgeonnement (ou granulation) et la phase d'épithélialisation. Tout au long du processus de cicatrisation, la plaie produit des exsudats fluides ou visqueux qui doivent si possible être absorbés par un pansement cicatrisant ou évacués par ce dernier pour être guidés vers un réservoir à l'extérieur de la plaie (cas de la thérapie par pression négative).

Suivant la gravité de la plaie, le processus de cicatrisation peut s'étendre de quelques jours à plusieurs mois. Dans le cas de plaies particulièrement exsudatives, les exsudats fluides ou visqueux peuvent inonder le lit de la plaie et constituer un environnement favorable à la dégradation des tissus sains péri-lésionnels, du fait de la macération des tissus ou de leur surinfection.

Pour ce type de plaies, le rôle d'un pansement est donc d'absorber ou de gérer ces exsudats fluides pour limiter la macération, tout en restant en contact avec la plaie tout au long du processus de cicatrisation, afin de garantir une protection de la plaie vis-à-vis de l'environnement extérieur.

La réalisation d'un pansement doit ainsi remplir un cahier des charges complexe et concilier des caractéristiques contradictoires. En particulier, le pansement doit présenter une bonne respirabilité tout en évitant les risques de fuites et de macération, être imperméable aux liquides et aux bactéries tout en étant respirant (c'est-à-dire perméable à la vapeur d'eau), garder sa cohésion quand on le retire, et être facile à fabriquer. Le pansement doit également être facile à poser et rester en place le plus longtemps possible au cours du temps sans altérer la peau périlésionnelle, présenter une capacité d'absorption des exsudats élevée, et ne pas altérer la cicatrisation de la plaie lors de son retrait. Le pansement doit être enfin compatible avec l'utilisation complémentaire d'un système de contention.

Pour favoriser une partie de la gestion des fluides à travers le pansement, il est connu de mettre en oeuvre dans ces derniers différentes couches pouvant notamment être constituées de matériaux polymériques et présentant une perméabilité à la vapeur d'eau (MVTR) élevée. On estime en effet qu'au cours d'une journée, la perspiration de la peau génère une perte insensible d'eau d'environ 250 g/m$^2$ [B. Gabard dans l'encyclopédie Médico-Chirurgicale, 50-140-E-10]. Ainsi, pour être considéré comme efficace, un dispositif médical dans son ensemble doit donc présenter une perméabilité à la vapeur d'eau (MVTR) supérieure à cette valeur.

On cherche ainsi toujours à optimiser la perméabilité à la vapeur d'eau de chacune des couches constitutives d'un pansement. En particulier, le développement de la respirabilité de la matrice adhésive (également appelée masse adhésive), constituant généralement une couche de contact intermédiaire avec la peau, revêt un intérêt tout particulier. Néanmoins, l'amélioration de la respirabilité de la matrice adhésive s'accompagne généralement d'une diminution très importante du pouvoir adhésif de ladite matrice sur la peau. En effet, pour favoriser la perméabilité à la vapeur d'eau, il est d'usage d'ajouter la matrice, ce qui limite la surface de contact de cette dernière avec la peau, diminuant ainsi son pouvoir adhésif.

La demande WO 2008/074333 de la société Coloplast décrit d'améliorer la perméabilité de masses adhésives et décrit une matrice élastomérique pour dispositif médical comprenant un élastomère tribloc de type S-I-S et un sel hydrosoluble afin d'augmenter la perméabilité à la vapeur d'eau à des niveaux de 20 g/m²/24h. La demande WO 2015/192122 divulgue une composition comprenant au moins un adhésif, un agent gélifiant et un agent non gélifiant.

On demeure néanmoins à la recherche de compositions pour matrices adhésives entrant dans la constitution de tout dispositif, et notamment de tout dispositif à visée médicale, qui présentent des propriétés améliorées de perméabilité à la vapeur d'eau, tout en conservant un pouvoir adhésif suffisant.

La présente invention propose une composition, notamment susceptible d'être mise en oeuvre sous la forme d'une matrice adhésive permettant de répondre à l'ensemble de ces problématiques, ainsi qu'un dispositif médical la mettant en oeuvre.

## RESUME DE L'INVENTION

Ainsi, la présente invention a permis de mettre au point une composition spécifique à base d'au moins un adhésif acrylique et de particules d'un polymère réticulé ayant une densité de groupement carboxylate comprise entre 2,0 et 12,0 meq/g et une taille de pore moyenne comprise entre 0,005 et 1,0 $\mu$m, permettant la préparation d'une matrice adhésive susceptible d'être mise en oeuvre dans un dispositif, de préférence un dispositif médical tel qu'un pansement, ladite matrice présentant une très bonne perméabilité à la vapeur d'eau (MVTR), tout en conservant un pouvoir adhésif (PA) suffisant.

Plus précisément, il a été découvert, et ceci constitue le fondement de la présente invention, qu'en associant, dans une composition, au moins un adhésif acrylique avec des particules spécifiques d'un polymère réticulé ayant une densité de groupement carboxylate particulière et une taille de pore elle aussi particulière, il était possible, de façon tout à fait surprenante, d'optimiser la diffusion de la vapeur d'eau au travers de la composition, notamment lorsque celle-ci est mise en oeuvre sous forme de matrice adhésive. L'amélioration des propriétés de perméabilité à la vapeur d'eau de la composition permet, lorsqu'elle est mise en oeuvre dans un dispositif médical tel qu'un pansement, d'optimiser le taux d'humidité au niveau de la peau, favorisant ainsi une meilleure cicatrisation. Ainsi selon un premier aspect, la présente invention a pour objet une composition, notamment utile pour la fabrication de pansements, comprenant:

- pour 100 parties en poids, d'au moins un adhésif acrylique,
- de 0,01 à 25 parties en poids, de particules d'un polymère réticulé ayant une densité de groupement carboxylate comprise entre 2,0 et 12,0 meq/g et une taille de pore moyenne comprise entre 0,005 et 1,0 $\mu$m.

## DESCRIPTION DETAILLEE

### Polymère adhésif

La composition selon l'invention comprend au moins un adhésif acrylique.

De préférence, l'adhésif acrylique mis en oeuvre dans les compositions de l'invention est physiologiquement acceptable pour la peau, et sa structure est au moins partiellement réticulée, de préférence totalement réticulée.

Parmi les adhésifs convenant à la présente invention, on distingue les adhésifs respectant la peau (de l'anglais « soft skin adhesives » (SSA)) et les adhésifs sensibles à la pression (de l'anglais « pressure sensitive adhesives » (PSA)).

Selon un mode de réalisation préféré de l'invention, l'adhésif acrylique est un adhésif sensible à la pression (PSA).

Selon un mode de réalisation préféré de l'invention, l'adhésif acrylique est un polymère d'adhésif acrylique mis en oeuvre sous forme de solution, ou sous la forme d'une suspension. De préférence, le polymère d'adhésif acrylique est mis en oeuvre sous la forme de suspension aqueuse.

Lorsque l'adhésif acrylique est introduit dans la composition selon l'invention sous forme de suspension, de préférence sous forme de suspension aqueuse, ladite suspension présentant entre 40 et 80% de polymère adhésif acrylique, par rapport au poids total de la suspension.

Selon un mode de réalisation préféré, le polymère d'adhésif acrylique sensible à la pression est préparé à partir d'au moins un monomère d'acrylate d'alkyle.

Le monomère d'acrylate d'alkyle convenant à la réalisation de l'adhésif acrylique selon l'invention est notamment choisi parmi les monomères suivants : l'acrylate d'isooctyle, l'acrylate de 2-éthylhexyle, l'acrylate de butyle, l'acrylate de sec-butyle, le méthyle butyle acrylate, le 4-methyl 2-pentyl acrylate, l'acrylate de vinyle et de leurs dérivés.

En particulier, le polymère d'adhésif acrylique peut contenir de 30% à 98% en poids, d'au moins un monomère d'acrylate d'alkyle. A titre d'exemple, la concentration totale en acrylate d'alkyle est comprise entre 60 et 85% en poids par rapport au poids total du monomère.

Des comonomères peuvent être utilisés dans le cadre de la présente invention. Parmi ces comonomères, on peut citer de façon non limitative les acides carboxyliques ou dicarboxyliques insaturés tels que l'acide méthacrylique, l'acide acrylique, l'acide fumarique, le dibutyl fumarate, le dioctyl maleate et leurs dérivés.

D'autres comonomères peuvent être utilisés tels que les méthacrylates comme le méthacrylate de methyle, l'isodecyl méthacrylate ou d'autres variantes, les styrenes, la vinyl pyrolidone, et leurs dérivés.

Parmi les exemples d'adhésifs acryliques sensibles à la pression disponibles commercialement, on retrouve les adhésifs commercialisés sous la marque Gelva GME® par Cytec puis Henkel, Aeroset® par Ashland, Novacryl® par Omnova, Flexcryl® par Air Products, Robond® par Dow, Duro-Tak® par Henkel, ou encore Aquence® PS par Henkel également.

De façon préférée, le polymère acrylique adhésif sensible à la pression utilisé dans le cadre de la présente invention est celui commercialisé sous la référence Aquence® PS 6084-33 par la société HENKEL.

La composition selon l'invention comprend de 80 à 99,99% en poids, par rapport au poids total de la composition, d'au moins un adhésif acrylique.

Particules de polymère réticulé

Les compositions selon l'invention comprennent des particules d'un polymère réticulé ayant une densité de groupement carboxylate comprise entre 2,0 et 12,0 meq/g et une taille de pore moyenne comprise entre 0,005 et 1,0 $\mu$m.

La « taille moyenne des pores » désigne une taille moyenne exprimée en volume, dont la valeur peut être calculée par la formule 4 V/S où S est la surface spécifique et V est le volume de pores par unité de masse obtenu à partir d'une distribution de taille de pore mesurée par la méthode de compression au mercure.

La taille moyenne des pores, exprimée en volume, peut être déterminée par toute méthode connue de l'homme du métier, par exemple par porosimétrie à intrusion de mercure ou sorptométrie par adsorption d'azote.

Par exemple, la sorptométrie par adsorption d'azote peut être réalisée au moyen d'un appareil TriStar II Micromeritics couplé à un Smart VacPrep Micromeritics. Le lot à caractériser est par exemple soumis à une phase de dégazage pendant 24 heures à température ambiante puis 5 heures à 50°C. La température en cours d'essai est de -196°C, et la pression maintenue dans une gamme de $0 < P/P_0 < 0,30$ avec $P_0$ = pression de vapeur saturante de l'azote.

La porosimétrie à intrusion de mercure peut être mise en oeuvre au moyen d'une cellule de mesure pour poudre ayant un volume de 3cm$^3$ et un capillaire de volume de 0,387cm$^3$. L'analyse est réalisée en deux temps : dans un premier temps l'ensemble « pénétromètre-échantillon » est en configuration « basse pression » (mesure de 0,52 psia (vide primaire) jusqu'à 30 psia soit 2 bars) ; dans un second temps l'ensemble « pénétromètre-échantillon » est en configuration « haute pression » (mesure jusqu'à 60000psia soit 4000 bars). La taille minimale des pores accessibles est de 3 mm.

Selon un mode préféré de réalisation, les particules de polymère réticulé mises en oeuvre dans le cadre de la présente demande présentent une surface spécifique inférieure à 1 m$^2$/g. La surface spécifique peut notamment être mesurée par la méthode BET d'adsorption physique, bien connue de l'homme du métier.

Selon un mode préféré de réalisation, le polymère mis en oeuvre est un polymère organique.

Le polymère mis en oeuvre présente une densité de groupement carboxylate comprise entre 2,0 et 12,0 meq/g. Le groupement carboxylate est un groupe polaire conférant au polymère les propriétés d'absorption d'humidité souhaitées.

Il n'y a pas de limitation particulière quant à la nature du sel mis en oeuvre pour la formation des groupements carboxylate. Il peut par exemple s'agir d'un sel de métal alcalin tel que Li, Na, K, Rb et Cs, de métal alcalino-terreux tel que Be, Mg, Mg, Ca, Sr et Ba, d'autres métaux tels que Cu, Zn, Al, Al, Mn, Ag, Fe, Co et Ni, NH4 et des cations organiques tels que des amines.

De préférence, le sel de carboxylate utilisé dans le cadre de la présente invention est le carboxylate de sodium.

L'introduction de groupements carboxylates peut être effectuée par toute méthode connue de l'homme du métier. Par exemple, un monomère porteur d'un groupement carboxylate peut être homopolymérisé ou copolymérisé avec d'autres monomères pour donner le polymère selon l'invention. Alternativement, un polymère porteur de groupements carboxylés peut être salifié. Alternativement encore, un polymère peut d'abord être greffé par des groupements carboxylés, lesquels seront ensuite salifiés. Ces méthodes d'introduction de groupements carboxylates sont décrites en détails dans la demande de brevet US 6 080 797.

Un exemple typique de particules de polymère réticulé selon l'invention peut être réalisé à partir d'acrylonitrile ou d'acide méthacrylique.

Plus particulièrement, des microparticules de polymère réticulé, en particulier de polyacrylonitrile, selon l'invention peuvent être obtenues par coagulation ou par polymérisation par précipitation pour donner un agglomérat de particules de polyacrylonitrile ou un polymère d'acrylonitrile, cet agglomérat ou ce polymère subit une réticulation avec de l'hydrazine ou un dérivé d'hydrazine et enfin une hydrolyse au moins partielle des groupements nitrile résiduels de sorte à obtenir une densité de groupements carboxylate comprise entre 2,0 et 12,0 meq/g. En outre les différentes étapes de ce procédé permettent d'obtenir une taille de pore moyenne comprise entre 0,005 et 1,0 $\mu$m.

A titre illustratif, une première méthode permettant la fabrication d'un polymère réticulé selon l'invention consiste à préparer une solution de polymère à partir d'un polymère acrylonitrile et d'un solvant, à faire ensuite coaguler ladite solution dans un solvant qui n'est pas un solvant pour ledit polymère acrylonitrile pour obtenir un polymère acrylonitrile poreux, puis faire réticuler ledit polyacrylonitrile poreux avec une hydrazine, ladite réticulation étant suivie finalement d'une hydrolyse des groupements nitriles résiduels de manière à obtenir une densité de groupement carboxylate comprise entre 2,0 et 12,0 meq/g et une taille de pore moyenne comprise entre 0,005 et 1,0 $\mu$m.

Alternativement, une seconde méthode permettant la fabrication d'un polymère réticulé selon l'invention consiste à faire polymériser par précipitation un mélange de monomères contenant au moins 50% en poids d'acrylonitrile pour obtenir un polymère acrylonitrile poreux, puis réticulation dudit polyacrylonitrile poreux avec une hydrazine et hydrolyse des groupements nitriles résiduels de manière à obtenir une densité de groupement carboxylate comprise entre 2,0 et 12,0 meq/g et une taille de pore moyenne comprise entre 0,005 et 1,0 $\mu$m.

Ces procédés sont décrits plus précisément dans la demande de brevet US 6 080 797.

Néanmoins, toutes particules ayant les propriétés de densité de groupement carboxylate ou de taille de pore requises conviennent à la réalisation d'une composition selon l'invention. Une variante typique consacrerait par exemple la réalisation de telles particules à partir d'acide méthacrylique telles que décrites à l'exemple 5 du brevet US 6 080 797.

Il n'y a pas de limitation particulière quant à la forme des particules de polymère mises en oeuvre selon l'invention.

Au sens de la présente invention, le polymère réticulé selon l'invention est caractérisé par une humidité relative d'équilibre (mesurée à 20°C sous une atmosphère à 65% d'humidité relative) comprise entre 20 et 80%, de préférence entre 30 et 70%.

Selon un mode particulier de réalisation, les particules de polymère réticulé selon l'invention présentent une taille moyenne comprise entre 0,1 et 100$\mu$m, de préférence entre 0,3 et 64$\mu$m.

Selon un autre mode particulier de réalisation, les particules d'un polymère réticulé selon l'invention présentent une masse volumique apparente comprise entre 0,1 et 1 g/cm$^3$, de préférence entre 0,2 et 0,7 g/cm$^3$.

De telles particules sont par exemple commercialisées par la société Japan Exlan Co., Ltd sous la dénomination Taftic® HU 707E, Taftic® HU 720SF ou Taftic® HU 1200P.

Elles peuvent être introduites dans les compositions selon l'invention sous forme de poudre, ou sous forme de particules en suspension ou en dispersion dans de l'eau.

La composition selon l'invention comprend entre 0,01 et 25 parties en poids, pour 100 parties en poids d'au moins un adhésif acrylique, d'un polymère réticulé ayant une densité de groupement carboxylate comprise entre 2,0 et 12,0 meq/g et une taille de pore moyenne comprise entre 0.005 et 1.0 $\mu$m, de préférence de 0,03 à 15 parties en poids.

Actifs

Outre les particules de polymère réticulé ayant une densité de groupement carboxylate comprise entre 2,0 et 12,0 meq/g et une taille de pore moyenne comprise entre 0,005 et 1,0 $\mu$m, la composition selon l'invention peut comprendre une (ou plusieurs) autre(s) substance(s) active(s) permettant d'induire ou d'accélérer la cicatrisation ou pouvant avoir un rôle favorable dans le traitement cutané.

Parmi ces substances actives, on peut citer, en particulier, à titre d'exemples:

- les agents favorisant la cicatrisation tels que le rétinol, la vitamine A, la vitamine E, la N-Acétyl Hydroxyproline, les extraits de Centella Asiatica, la papaïne, les huiles essentielles de thym, niaouli, romarin, sauge, l'acide hyaluronique, le sucrose octasulfate de potassium, le sucralfate, l'allantoïne.
- les agents antibactériens tels que les sels ou complexes d'argent (tels les sulfates d'argent, les nitrates d'argent, les sulfamides d'argent ou encore les zéolites à base d'argent), les sels de zinc ou de cuivre, le métronidazole, la néomycine, les pénicillines, l'acide clavulanique, les tétracyclines, la mynocycline, la chlorotétracycline, les aminoglycosides, l'amikacine, la gentamicine, les probiotiques ;
- les antiseptiques tels que la chlorhexidine, le trichlosan, le biguanide, l'hexamidine, le thymol, le lugol, la povidone iodée, le chlorure de benzalkonium et de benzethonium ;
- les anti-douleurs tels que le paracétamol, la codéine, le dextropropoxyphène, le tramadol, la morphine et ses dérivés, les corticoïdes et leurs dérivés ;
- les anesthésiques locaux tels que la lidocaïne, la benzocaïne, la dibucaïne, le chlorhydrate de pramoxine, la bupivacaïne, la mépivacaïne, la prilocaïne, l'étidocaïne ;
- les anti-inflammatoires comme les anti-inflammatoires non stéroïdiens (AINS), l'aspirine ou acide acétylsalicylique, l'ibuprofène, le kétoprofène, le flurbiprofène, le diclofenac, l'acéclophénac, le kétorolac, le méloxicam, le piroxicam, le ténoxicam, le naproxène, l'indométacine, le naproxcinod, le nimésulid, le célécoxib, l'étoricoxib, le parécoxib, le rofécoxib, le valdécoxib, la phénylbutazone, l'acide niflumique, l'acide méfénamique.

Ces agents actifs pourront être utilisés en une quantité de l'ordre de 0,01 à 25 parties en poids, de préférence de 1 à 21 parties en poids, pour 100 parties en poids d'au moins un adhésif acrylique.

Bien entendu, la composition selon l'invention peut aussi comprendre un ou plusieurs autres composés connus pour leur action dans la phase de détersion comme par exemple :

- des enzymes ;
- l'urée.

Matrice adhésive

Afin de réaliser tout dispositif, et de préférence un pansement, les compositions selon l'invention seront de préférence mises en forme par enduction puis séchage, pour former une matrice adhésive.

L'invention a ainsi pour objet, selon un autre aspect, une matrice adhésive obtenue à partir d'une composition selon l'invention telle que décrite précédemment.

Cette matrice peut être continue, c'est-à-dire pleine et ne présentant pas la moindre perforation, ou discontinue, c'est-à-dire présentant au moins une perforation ou des trous traversants, de préférence, ladite matrice adhésive est continue.

En présence de trous traversants, ces derniers peuvent être réalisés par perforation, évidage ou poinçonnage d'une composition selon l'invention préalablement formée en couche mince, seule ou associée à un support provisoire.

Les trous traversants peuvent être de géométrie quelconque et présenteront par exemple une section transversale circulaire, rectangulaire, trapézoïdale ou carrée.

Ces trous seront répartis, de préférence de façon régulière, avec une densité telle que la surface totale des trous représente entre 20 et 70 %, et de préférence entre 30 et 60 % de la surface totale de la matrice.

D'une façon générale, les matrices adhésives conformes à l'invention présenteront une épaisseur comprise entre 0,1 $\mu$m et 2 mm, et de préférence entre 5 $\mu$m et 150 $\mu$m.

Il peut également être envisagé d'utiliser cette matrice adhésive pour enduire une armature ou un support.

Dispositif

L'invention a ainsi pour objet, selon un mode préféré de réalisation, un dispositif médical tel qu'un patch, un film, une bande ou encore un pansement, ou bien un dispositif de type textile fonctionnel tel qu'un article de sport, comprenant une matrice adhésive telle que décrite précédemment. De préférence, ledit dispositif est un pansement caractérisé en ce qu'il comprend une matrice adhésive telle que précédemment décrite.

Selon un mode de réalisation préféré, la présente demande vise à couvrir un pansement comprenant une matrice adhésive se présentant sous la forme d'une couche mince obtenue à partir d'une composition comprenant :

- pour 100 parties en poids d'au moins un adhésif acrylique,
- de 0,01 à 25 parties en poids de particules d'un polymère réticulé ayant une densité de groupement carboxylate comprise entre 2,0 et 12,0 meq/g et une taille de pore moyenne comprise entre 0,005 et 1,0 $\mu$m

La matrice adhésive peut être utilisée seule pour constituer le dispositif ou en association avec une ou plusieurs couches, telles que des mousses, des textiles, des matériaux composites ou encore des films.

La matrice adhésive peut se trouver à n'importe quel niveau du dispositif, c'est-à-dire constituer l'une quelconque des différentes couches du dispositif, et peut être en contact avec la peau, la plaie.

La présente invention est illustrée dans les exemples non limitatifs présentés ci-après.

**Exemples**

Préparation des compositions

Les compositions 1 à 3 ont été élaborées à l'aide des constituants suivants dans les proportions, exprimées en parties en poids, mentionnées dans le tableau 1 ci-dessous.

Fabrication de la composition

Dans un dispositif de mélange placé sous agitation, on ajoute à une suspension d'une base Aquence PS 6084-33, une quantité déterminée de particules de Taftic HU sous forme de poudre ou en suspension aqueuse jusqu'à l'obtention d'un mélange homogène.

Adhésif acrylique :

- Aquence PS 6084-33 de Henkel

Particules de polymère réticulé:

- Taftic HU-720SF de la société Japan Exlan Co., Ltd
- Taftic HU-1200P de la société Japan Exlan Co., Ltd
- Taftic HU-707E de la société Japan Exlan Co., Ltd

Tableau 1 :

|  | Compo. 1 | Compo. 2 | Compo. 3 | Contrôle |
|---|---|---|---|---|
| Taftic HU-720 SF de Japan Exlan Co., Ltd | 4.16 |  |  |  |
| Taftic HU-1200 P de Japan Exlan Co., Ltd |  | 4.16 |  |  |
| Taftic HU-707 E de Japan Exlan Co., Ltd |  |  | 20,48 |  |
| Aquence PS 6084-33 de Henkel | 100 | 100 | 100 | 100 |

Mesure de la perméabilité à la vapeur d'eau :
Les conditions de réalisation du test se basent sur la norme NF EN 13726 - liquides en contact.

MATERIEL / APPAREILLAGE

Balance résolution 0,1 mg
Emporte-pièce diamètre 44mm,
Cellule de mesure en aluminium de diamètre D = 35,7 mm et surface 10 $cm^2$
Fiole jaugée (= 25mL) ou pompe doseuse
Etuve thermostatée à 37°C et < 20% d'humidité relative (HR) à brassage d'air
Pièce climatisée à 21°C +/-2 - HR à 60% +/- 15
Réactifs : eau déminéralisée et solution NaCl, $CaCl_2$.

ECHANTILLONNAGE/ CONDITIONNEMENT DES ECHANTILLONS

Nombre d'éprouvette n $\geq$ 5.
Température T = 37°C $\pm$ 2 °C.
Hygrométrie HR < 20%

MODE OPERATOIRE

Les conditions de réalisation du test se basent sur la norme NF EN 13726 - liquides en contact, étaient les suivantes.
Le principe de cette mesure est le suivant :

1. Verser à l'intérieur de la cellule un volume V de liquide
2. Positionner, sur l'ouverture de la cellule de mesure, l'échantillon à tester (face adhésive sur le joint siliconé si le produit est adhésivé)
3. Positionner par-dessus l'échantillon, centré pour éviter les fuites, le dispositif de maintien puis visser les 3 vis jusqu'en butée
4. Peser l'ensemble $PMVTR_0$
5. Retourner les cellules pour mettre le liquide en contact avec l'échantillon. Placer cet ensemble dans une étuve à une température T pendant un temps t (t exprimé en heure)

6. Peser dans les 5 minutes suivant la fin du test (t) l'ensemble du dispositif PMVTR1

EXPRESSION DES RESULTATS

Calculer le taux de transmission à la vapeur d'eau (MVTR)

$$MVTR_1 = (PMVTR_0 - PMVTR_1)/(\pi D^2/4)$$

$$MVTR_1 = 4 \, (PMVTR_0 - PMVTR_1)/\pi D^2$$

Soit, dans les conditions standard, $MVTR_1 = (PMVTR_0 - PMVTR_1)/(10*10^{-4})$

Exprimer le résultat en $g/m^2/24h$.

Calculer la moyenne des n essais, au $g/m^2$ près.

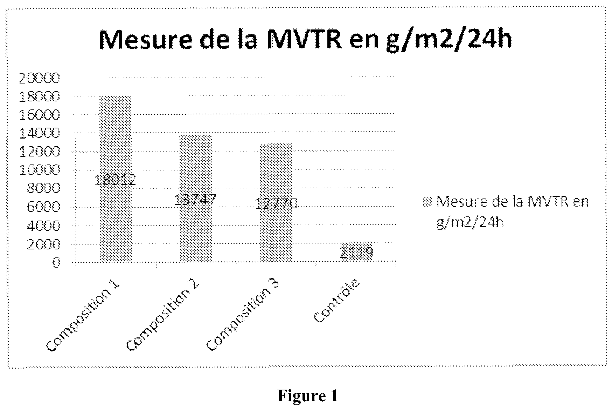

Les résultats des mesures de perméabilité à la vapeur d'eau sont présentés à la Figure 1 et au tableau 1 suivant. Ces résultats montrent que chacune des trois compositions comprenant des particules d'un polymère réticulé selon l'invention, présentent une capacité d'absorption de la vapeur d'eau significativement supérieure à la composition contrôle.

Tableau 1 :

|  | Valeurs de MVTR en $g/m^2$ 24h |
|---|---|
| Composition 1 | 18012 +/- 538 |
| Composition 2 | 13747 +/- 3265 |
| Composition 3 | 12770 +/- 3061 |
| Contrôle | 2119 +/- 741 |

**Mesure du pouvoir adhésif à 90°**

La première étape consiste à apposer l'une des compositions de l'exemple sur un support de type non tissé et à découper ensuite une éprouvette c'est-à-dire une bande de l'une de ces associations de largeur 20 mm et de longueur 300 mm.

Dans une deuxième étape, on applique l'éprouvette, avec une légère pression du doigt sur la plaque d'acier parallèlement à sa plus grande dimension, sans étirer la bande et en évitant d'inclure la moindre bulle d'air.

On fait deux allers retours à l'aide d'un rouleau applicateur, à une vitesse V1 = 10 mm/s et un poids P = 2 kg/cm, sans pression additionnelle, pour obtenir un contact intime entre l'adhésif acrylique et la plaque d'acier.

On laisse l'ensemble climatiser à une température de 23+/-2°C pendant un temps t = 10 min.

Enfin, dans une étape terminale, on enregistre la force moyenne F du pelage à 90° réalisé à une vitesse V2 = 300 mm/min de l'éprouvette sur la plaque d'acier au moyen d'un dynamomètre électronique.

|  | Pouvoir adhésif à 90° sur plaque d'acier (cN/cm) |
|---|---|
| Composition 1 | 278 +/- 35 |
| Contrôle | 286 +/- 22 |

Ainsi, l'ajout de particules d'un polymère réticulé ayant une densité de groupement carboxylate comprise entre 2,0 et 12,0 meq/g et une taille de pore moyenne comprise entre 0,005 et 1,0 $\mu$m permet ainsi d'augmenter significativement la capacité d'absorption de la vapeur d'eau des compositions selon l'invention, sans altérer son pouvoir adhésif.

**Revendications**

**1.** Composition comprenant:

- pour 100 parties en poids, d'au moins un adhésif acrylique,
- de 0,01 à 25 parties en poids, de particules d'un polymère réticulé ayant une densité de groupement carboxylate comprise entre 2,0 et 12,0 meq/g et une taille de pore moyenne comprise entre 0,005 et 1,0 $\mu$m, déterminées comme indiqué dans la description.

2. Composition selon la revendication 1, **caractérisée en ce que** l'adhésif acrylique se présente sous forme de solution ou de suspension, de préférence sous forme de suspension aqueuse.

3. Composition selon la revendication 2, **caractérisée en ce que** ladite suspension présente entre 40 et 80% de polymère adhésif acrylique, par rapport au poids total de la suspension.

4. Composition selon la revendication 3, **caractérisée en ce que** le polymère acrylique adhésif est préparé à partir d'au moins un monomère d'acrylate d'alkyle, notamment choisi parmi les monomères d'acrylate d'isooctyle, d'acrylate de 2-éthylhexyle, d'acrylate de butyle, d'acrylate de sec-butyle, du méthyle butyle acrylate, du 4-methyl 2-pentyl acrylate, ou d'acrylate de vinyle.

5. Composition selon l'une des revendications précédentes, **caractérisée en ce que** les particules de polymères réticulées sont introduites dans ladite composition sous forme de poudre, de dispersion ou de suspension, de préférence sous forme de poudre.

6. Composition selon l'une des revendications précédentes, **caractérisée en ce que** les particules de polymères réticulées présentent une taille moyenne comprise entre 0,1 et 100 $\mu$m, de préférence entre 0,3 et 64 $\mu$m, déterminée comme indiqué dans la description.

7. Composition selon l'une des revendications précédentes, **caractérisée en ce que** le polymère réticulé est préparé à partir d'acrylonitrile ou d'acide méthacrylique.

8. Composition selon l'une des revendications précédentes, **caractérisée en ce qu'**elle comprend une ou plusieurs substances active(s) permettant d'induire ou d'accélérer la cicatrisation ou pouvant avoir un rôle favorable dans le traitement cutané.

9. Matrice adhésive **caractérisée en ce qu'**elle est obtenue à partir d'une composition selon l'une des revendications 1 à 8, de préférence par enduction et séchage.

10. Dispositif médical **caractérisé en ce qu'**il comprend une matrice adhésive selon la revendication 9.

11. Dispositif médical selon la revendication 10 **caractérisé en ce qu'**il s'agit d'un pansement.

**Patentansprüche**

1. Zusammensetzung, welche umfasst:

- 100 Gewichtsteile wenigstens eines Acrylhaftstoffs,
- 0,01 bis 25 Gewichtsteile von Teilchen eines vernetzten Polymers mit einer Carboxylatgruppendichte zwischen 2,0 und 12,0 meq/g und einer mittleren Porengröße zwischen 0,005 und 1,0 $\mu$m, bestimmt wie in der Beschreibung angegeben.

2. Zusammensetzung nach Anspruch 1, **dadurch gekennzeichnet, dass** das Acrylhaftmittel in Form einer Lösung oder Suspension, bevorzugt in Form einer wässrigen Suspension, vorliegt.

3. Zusammensetzung nach Anspruch 2, **dadurch gekennzeichnet, dass** die Suspension zwischen 40 und 80 % Acrylhaftstoffpolymer, bezogen auf das Gesamtgewicht der Suspension, enthält.

4. Zusammensetzung nach Anspruch 3, **dadurch gekennzeichnet, dass** das Acrylhaftstoffpolymer aus wenigstens einem Alkylacrylatmonomer hergestellt ist, insbesondere gewählt aus den Monomeren Isooctylacrylat, 2-Ethylhexylacrylat, Butylacrylat, sec-Butylacrylat, Methylbutylacrylat, 4-Methyl-2-pentylacrylat oder Vinylacrylat.

**5.** Zusammensetzung nach einem der vorhergehenden Ansprüche, **dadurch gekennzeichnet, dass** die vernetzten Polymerteilchen in Form von Pulver, Dispersion oder Suspension, bevorzugt in Form von Pulver, in die Zusammensetzung eingebracht werden.

**6.** Zusammensetzung nach einem der vorhergehenden Ansprüche, **dadurch gekennzeichnet, dass** die vernetzten Polymerteilchen eine durchschnittliche Größe zwischen 0,1 und 100 $\mu$m, bevorzugt zwischen 0,3 und 64 $\mu$m, aufweisen, bestimmt wie in der Beschreibung angegeben.

**7.** Zusammensetzung nach einem der vorhergehenden Ansprüche, **dadurch gekennzeichnet, dass** das vernetzte Polymer aus Acrylnitril oder Methacrylsäure hergestellt ist.

**8.** Zusammensetzung nach einem der vorhergehenden Ansprüche, **dadurch gekennzeichnet, dass** diese einen oder mehrere Wirkstoffe umfasst, die die Wundheilung induzieren oder beschleunigen können oder eine günstige Rolle bei der Hautbehandlung spielen können.

**9.** Haftmatrix, **dadurch gekennzeichnet, dass** diese aus einer Zusammensetzung nach einem der Ansprüche 1 bis 8, bevorzugt durch Beschichten und Trocknen, hergestellt ist.

**10.** Medizinische Vorrichtung, **dadurch gekennzeichnet, dass** sie eine Haftmatrix nach Anspruch 9 umfasst.

**11.** Medizinische Vorrichtung nach Anspruch 10, **dadurch gekennzeichnet, dass** es sich um einen Verband handelt.

**Claims**

**1.** Composition comprising:

- for 100 parts by weight of at least one acrylic adhesive,
- from 0.01 to 25 parts by weight of particles of a cross-linked polymer having a carboxylate-group density between 2.0 and 12.0meq/g and an average pore size between 0.005 and 1.0$\mu$m determined as indicated in the specification.

**2.** Composition according to claim 1, **characterised in that** the acrylic adhesive is in the form of a solution or a suspension, preferably in the form of an aqueous suspension.

**3.** Composition according to claim 2, **characterised in that** said suspension has between 40 and 80% of acrylic adhesive polymer, relative to the total weight of the suspension.

**4.** Composition according to one of the previous claims, **characterised in that** the adhesive acrylic polymer is prepared from at least one monomer of alkyl acrylate, in particular chosen from the monomers of isooctyl acrylate, 2-ethylhexyl acrylate, butyl acrylate, sec-butyl acrylate, methyl butyl acrylate, 4-methyl 2-pentyl acrylate, or vinyl acrylate.

**5.** Composition according to one of the previous claims, **characterised in that** the particles of cross-linked polymer are introduced in the composition in the form of a powder, a dispersion or a suspension, preferably in the form of a powder.

**6.** Composition according to one of the previous claims, **characterised in that** the particles of cross-linked polymer have an average size between 0.1 and 100$\mu$m, preferably between 0.3 and 64$\mu$m.

**7.** Composition according to one of the previous claims, **characterised in that** the cross-linked polymer is prepared from acrylonitrile or methacrylic acid.

**8.** Composition according to one of the previous claims, **characterised in that** it comprises one or more active substance(s) allowing to induce or to accelerate healing or capable of playing a favourable role in skin treatment.

**9.** Adhesive matrix, **characterised in that** it is obtained from a composition according to one of claims 1 to 8, preferably by coating and drying.

**10.** Medical device, **characterised in that** it comprises an adhesive matrix according to claim 9.

**11.** Medical device according to claim 10, **characterised in that** it is a dressing.

**Figure 1**

## RÉFÉRENCES CITÉES DANS LA DESCRIPTION

**Documents brevets cités dans la description**

- WO 2008074333 A
- WO 2015192122 A

- US 6080797 A

**Littérature non-brevet citée dans la description**

- **B. GABARD.** *l'encyclopédie Médico-Chirurgicale*